(19)

**Europäisches Patentamt**
**European Patent Office**
**Office européen des brevets**

(11) **EP 4 161 623 B1**

(12) **EUROPEAN PATENT SPECIFICATION**

(45) Date of publication and mention
of the grant of the patent:
**18.12.2024  Bulletin 2024/51**

(21) Application number: **21731467.3**

(22) Date of filing: **08.06.2021**

(51) International Patent Classification (IPC):
**A61B 5/374** (2021.01)   **A61B 5/38** (2021.01)
**A61B 7/02** (2006.01)   **A61B 7/04** (2006.01)
**A61M 21/00** (2006.01)   **A61B 5/00** (2006.01)
**A61B 7/00** (2006.01)

(52) Cooperative Patent Classification (CPC):
**A61M 21/00; A61B 5/374; A61B 5/38;**
**A61B 5/4812; A61B 7/003; A61B 7/023;**
A61B 7/026; A61B 7/04; A61M 2021/0027;
A61M 2205/3375; A61M 2205/3553;
A61M 2205/3592; A61M 2205/505; A61M 2205/52;
A61M 2209/088;                                    (Cont.)

(86) International application number:
**PCT/EP2021/065327**

(87) International publication number:
**WO 2021/250018 (16.12.2021 Gazette 2021/50)**

(54) **AN AUDIO SYSTEM FOR AUDITORY STIMULATION FOR TREATMENT OF NEUROLOGICAL,**
**PSYCHOLOGICAL OR PSYCHIATRIC DISORDERS**

AUDIOSYSTEM ZUR GEHÖRSTIMULATION ZUR BEHANDLUNG VON NEUROLOGISCHEN,
PSYCHOLOGISCHEN ODER PSYCHIATRISCHEN ERKRANKUNGEN

SYSTÈME AUDIO POUR UNE STIMULATION AUDITIVE POUR LE TRAITEMENT DE TROUBLES
NEUROLOGIQUES, PSYCHOLOGIQUES OU PSYCHIATRIQUES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **09.06.2020  DK PA202000670**

(43) Date of publication of application:
**12.04.2023  Bulletin 2023/15**

(73) Proprietors:
 • **T&W Engineering A/S**
   **3540 Lynge (DK)**
 • **Aarhus Universitet**
   **8000 Aarhus C (DK)**

(72) Inventors:
 • **HEMMSEN, Martin Christian**
   **3540 Lynge (DK)**
 • **KIDMOSE, Preben**
   **8000 Aarhus C (DK)**

 • **CHRISTENSEN, Christian Bech**
   **8000 Aarhus C (DK)**
 • **RANK, Mike Lind**
   **3540 Lynge (DK)**

(56) References cited:
   **US-A1- 2013 190 556     US-A1- 2019 388 020**

 • **DON MANUEL ET AL: "Input and Output**
   **Compensation for the Cochlear Traveling Wave**
   **Delay in Wide-Band ABR Recordings:**
   **Implications for Small Acoustic Tumor**
   **Detection", JOURNAL OF THE AMERICAN**
   **ACADEMY OF AUDIOLOGY, vol. 20, no. 02, 1**
   **February 2009 (2009-02-01), CA, pages 099 - 108,**
   **XP055836707, ISSN: 1050-0545, Retrieved from**
   **the Internet**
   **<URL:https://www.ncbi.nlm.nih.gov/pmc/article**
   **s/PMC3102764/pdf/nihms292916.pdf> DOI:**
   **10.3766/jaaa.20.2.3**

EP 4 161 623 B1

(52) Cooperative Patent Classification (CPC): (Cont.)
A61M 2210/0662; A61M 2230/06; A61M 2230/10;
A61M 2230/205; A61M 2230/60; A61M 2230/63

(52) Cooperative Patent Classification (CPC): (Cont.)
A61M 2230/205; A61M 2230/60; A61M 2230/63

**Description**

FIELD OF THE INVENTION

The present invention relates to an audio system.

BACKGROUND OF THE INVENTION

It has been suggested that a multitude of different defective health conditions may be at least alleviated using auditory stimulation.

As one example, WO-A1-2014162286 discloses that psychological, psychiatric and neurological disorders such as sleep disorders/insomnia, anxiety, panic attacks, depression, obsessive- compulsive disorders, schizophrenia, headaches/migraines, tinnitus, diplopia, dementia, autism spectrum, Alzheimer's Disease and Pick's disease may be treated using auditory stimulation.

Additionally, WO-A1-2014162286 also discloses that physical and psychological peak performance in healthy subjects may be improved using auditory stimulation.

In the paper: "Gamma Band Neural Stimulation in Humans and the Promise of a New Modality to Prevent and Treat Alzheimer's Disease", by McDermott et al, in Journal of Alzheimer's Disease 65 (2018) 363 - 392, it is suggested that the inconvenience related to the duration of external stimuli (such as sound) based treatments to prevent and treat Alzheimer's disease may be alleviated by being carried out during sleep.

However, in the paper "Potentials evoked by chirp-modulated tones: a new technique to evaluate oscillatory activity in the auditory pathway", by Artieda et al, in Clinical Neurophysiology 115 (2004), 699-709 it was found that the amplitude of the evoked potential was smaller during sleep (in stage 2) compared to being awake. At 40 Hz the evoked potential (i.e. the response) during sleep was only 1/6 of the response while being awake. Further relevant prior art documents are US 2019/388020 A1 and US 2013/190556 A1.

Thus, there is a need to alleviate the inconvenience of auditory stimulations and also a need to improve the efficiency of auditory stimulations.

SUMMARY OF THE INVENTION

It is therefore an object of the present invention to provide an improved audio system for alleviating psychological, psychiatric or neurological disorders or for improving physical and psychological peak performance.

The invention, in a first aspect, therefore, provides an audio system according to claim 1.

This provides an audio system that is advantageous with respect to at least efficiency and convenience of use.

Further advantageous features appear from the dependent claims.

Still other objects of the present invention will become apparent to those skilled in the art from the following description wherein the invention will be explained in greater detail.

BRIEF DESCRIPTION OF THE DRAWINGS

The invention will be readily understood from the following detailed description in conjunction with the accompanying drawings. The drawings and descriptions will be regarded as illustrative in nature and not as restrictive. In the drawings:

Fig. 1    shows a highly schematic and simplified block diagram of an audio system according to a first embodiment of the present invention;

Fig. 2    illustrates highly schematically an audio system according to a second embodiment of the present invention;

Fig. 3    illustrates highly schematically an audio system according to a third embodiment of the present invention; and

Fig. 4    illustrates highly schematically a method of operating an audio system according to an embodiment of the present invention.

DETAILED DESCRIPTION

In the present context an audio system may comprise a single audio device or comprise two audio devices (one device for the left and right ear of the user respectively).

In the present context the terms auditory stimulation and entrainment may be used interchangeably because the object of both is to induce specific neural oscillations. It is noted though that entrainment generally is a broader term that not necessarily is based on audio stimulus. Furthermore, it is noted that the terms auditory stimulation and audio stimulation may likewise be used interchangeably.

Additionally, in the present context the term "neurological deficiency" is construed to cover at least mild cognitive impairment, dementia and Alzheimer's disease.

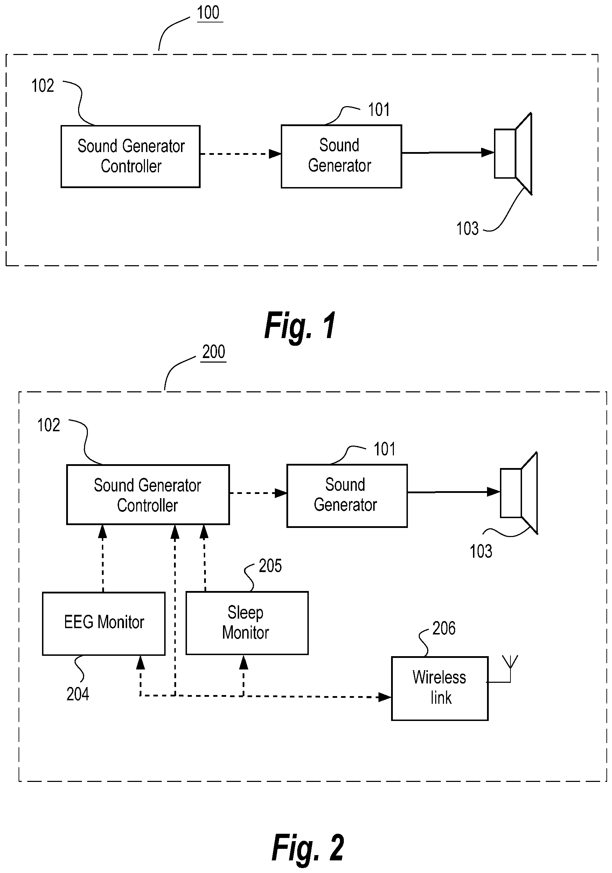

Reference is now made to Fig. 1, which illustrates highly schematically an audio system 100 according to a first embodiment of the invention. The audio system 100 comprises a sound generator 101, a sound generator controller 102, and an electrical-acoustical output transducer 103.

The sound generator controller 102 is configured to control the sound generator 101 and this involves at least determining the type of auditory stimulation to be provided by the electrical-acoustical output transducer 103 and when the auditory stimulation is to be initiated and terminated.

The specific characteristics, of a given type of auditory stimulation, are determined by a set of sound generator parameters. The specific characteristics include at least: the desired frequency of the neural oscillations to be induced and the volume of the auditory stimulation, which in the following may also be denoted the auditory stimulation level. In the following the volume or level of an auditory stimulation may be given in the units of dB nHL or dB SL (if hearing impaired persons are considered).

Generally, the sound generator 101 is configured to provide at least one type of auditory stimulation adapted to induce neural oscillations, in the frequency range between 0.5 and 100 Hz, in a brain region of a user of the audio system.

By inducing specific neural oscillations various neurological pathologies may be alleviated.

According to a more specific embodiment the sound generator 101 is configured to induce neural oscillations of 40 Hz or in the range between 35 - 45 Hz or even in the range between 32 - 48 Hz. Inducing neural oscillations at this frequency or in this range may be able to alleviate mild cognitive impairment, dementia and Alzheimer's disease.

According to various embodiments said neural oscillations are induced by providing an auditory stimulation comprising clicks or other forms of pulses that are repeatedly provided with a frequency (which in the following may also be denoted repetition frequency) that is the same as the frequency of the neural oscillations to be induced.

Furthermore, the individual pulses of the auditory stimulation are, according to the present embodiment, adapted to compensate the cochlear dispersion (i.e. to compensate for the frequency dependent travel time through the cochlea).

Auditory stimulation adapted to compensate the cochlear dispersion has been suggested for use in testing hearing based on recording auditory evoked responses. Such types of auditory stimulation have been disclosed e.g. in the paper "Auditory brainstem responses with optimized chirp signals", by Dau et al., in Journal of Acoustical Society America 107 (2000) 1530 - 1540.

However, it is a specific advantage of the present invention that auditory stimulation adapted to alleviate a neurological pathology by inducing neural oscillations is also adapted to compensate cochlear dispersion because the strength (which in the following may also be denoted level) of the induced neural oscillations is improved and hereby also the efficiency of treatments directed at alleviating neurological pathologies.

According to one especially advantageous aspect the improved efficiency can be used to enable efficient treatments of a shorter duration or lower sound level. The shorter duration is especially advantageous with respect to treatment during sleep where the available time window for a given treatment may be limited, one example of this is treatments directed at improving slow wave sleep. The lower sound level is also especially advantageous with respect to treatment during sleep because it generally lowers the risk of awakening a user.

The cochlear dispersion compensated pulses are provided by adding a plurality of pure tones each with a specific relative phase delay adapted to compensate the cochlear dispersion and hereby providing a chirp capable of compensating the cochlear dispersion.

According to a more specific embodiment the specific relative phase delay is determined using the delay model given in the paper "Auditory steady-state responses to chirp stimuli based on cochlear traveling", by Elberling et al., in Journal of Acoustical Society America 122, 2772-2785:

$$\tau = k \cdot f^{-d}$$

wherein $\tau$ is the delay in seconds, f is the frequency in Hz and the constants k and d have the values k = 0.0920 and d = 0.4356.

Said plurality of pure tones are separated by a constant frequency difference that is the same as the frequency of the neural oscillations to be induced (i.e. the repetition frequency), whereby a strong neural response at the repetition frequency may be obtained.

According to another even more specific embodiment said plurality of pure tones comprise pure tone frequencies covering a very broad frequency spectrum from say 100 Hz to 25 kHz whereby a stronger response may be obtained compared to selecting pure tone frequencies from a more narrow frequency range wherefrom a limited number of pure tones can be selected.

It is generally advantageous, in the present context, to use broad spectrum sounds compared to narrow spectrum sounds due to the lower risk of being woken up.

According to yet another specific embodiment said plurality of pure tones are provided during the full time repetition period (i.e. the time period representing the inverse of the repetition frequency), whereby an especially strong neural response may be obtained. For some users this may be used to provide auditory stimulation at a lower level, which may be advantageous e.g. in case the auditory stimulation is provided during sleep where it is essential that the user does not wake up.

According to still another embodiment it is ensured that the frequencies of the selected pure tones are harmonics of the repetition frequency, which is advantageous in order to ensure that the phase relationship between the selected pure tones remains the same at the beginning of each repetition time period.

According to an embodiment it is ensured that the phase relationship at the beginning of each repetition time period is the same by applying a window function in the time domain. Considering more specifically a repetition time period of 25 milliseconds (corresponding to a repetition frequency of 40 Hz) then attack and release times can be selected to be say 0.2 milliseconds.

According to another embodiment said specific relative phase delay for each of said pure tones are dependent on the level of the auditory stimulation relative to the average behavioral hearing threshold level for a group of orthologically normal young adults. This level of the auditory stimulation is typically given in the unit "dB nHL".

The specific relative phase delay for each of said pure tones may be determined in accordance with the delay models given in the paper "A direct approach for the design of chirp stimuli used for the recording of auditory brainstem responses", by Elberling et al., in Journal of Acoustical Society America 128, 2955-2964.

Thus, according to one aspect a relatively low level auditory stimulation may be provided while still being able to induce a satisfactory neural response due to the stronger auditory evoked response that can be achieved by taking the auditory stimulation level into account. This may be advantageous with respect to keeping users from waking-up if the auditory stimulation is provided during sleep.

Thus, it is hypothesized that the level of the auditory evoked response is correlated with the therapeutic effect to be achieved. Therefore, it is advantageous to optimize the auditory stimulation (i.e. the stimulus) in order to maximize the auditory evoked response.

According to an embodiment an auditory evoked response (e.g. in the form of an auditory brainstem response) is provided, which is based on an auditory stimulation with a sound level of 20 dB nHL or in the range between say 15 and 25 dB nHL or even in the range between 10 and 50 dB nHL. In case the user has a hearing loss the sound level of the auditory stimulation is adapted to be in the same numerical range, i.e. the unit of dB nHL is replaced with the unit of dB SL.

According to an embodiment a hearing loss of the audio system user is taken into account by filtering with a linear phase filter (i.e. with constant group delay) the auditory stimulation signal with the inverse characteristic of a specific user's audiogram, whereby the filtered signal will have for that specific user the same auditory stimulation level (measured in dB SL) as the original auditory stimulation level for normal hearing persons (measured in dB nHL). According to another obvious embodiment the hearing loss is taken into account by filtering the auditory stimulation signal in an frequency filter bank and applying a hearing loss compensating gain to the provided frequency band signals before combining the hearing loss compensated frequency band signals to provide a hearing loss compensated auditory stimulation signal.

According to an embodiment a target sound level of the auditory stimulation (e.g. in the range given above) is selected and based here on a corresponding delay model is selected (i.e. a specific sound level dependent chirp as defined by the specific relative phases of the sum of pure tones) that enables a desired level of the auditory evoked response to be achieved with the lowest possible auditory stimulation level (i.e. the lowest possible sound level). Thus, generally, according to the invention, the specific relative phases may depend on the auditory stimulation level.

According to one more specific embodiment the auditory evoked responses are determined in-situ using either an EEG monitor integrated in an audio system according to the invention or using an EEG monitor incorporated in a device external to - but operationally connected with - the audio system according to the invention.

According to embodiments the EEG monitor may be adapted to provide at least one of: measure a level of induced neural oscillations at 40 Hz, to measure a level of slow wave sleep, to measure EEG data for determining at least one stage of sleep, and to measure EEG data for evaluating the efficiency with respect to alleviating a neurological pathology.

Reference is now made to Fig. 2, which illustrates highly schematically an audio system 200 according to a second embodiment of the invention. The audio system 200 according to the second embodiment distinguishes the audio system 100 according to the first embodiment in further comprising an electroencephalogram (EEG) monitor 204, a sleep monitor 205 and a wireless link 206. It is noted that the components that are present in both embodiments have retained their numbering.

Generally, methods for monitoring sleep are known, thus determination of sleep stages during sleep is also known. One example of such methods is e.g. disclosed in JP2011083393 A. Common for most methods is that the determination of a sleep stage is obtained by solving a classification problem (or using a more generic term a pattern recognition problem) based on e.g. measured EEG data, ECG data or breathing rhythm.

According to one specific embodiment the EEG monitor 204 provides measured EEG data to the sleep monitor 205, that based hereon determines at least one specific sleep stage. The determined sleep stage is subsequently provided to the sound generator controller 102, which is adapted to initiate and terminate at least one type of auditory stimulation in response to the determined sleep stage.

An audio system, according to the present embodiment, with means for detecting various sleep stages is advantageous because it enables at least one auditory stimulation to be carried out during sleep, while ensuring that important sleep stages such as especially the slow wave sleep is not disturbed.

According to one specifically advantageous aspect the audio system is configured to only initiate auditory stimulations after it has been detected that a first sleep cycle has finished. Hereby it is ensured that the most important (with respect to health benefits) slow wave sleep stage, which takes place as part of the first sleep cycle is not compromised (e.g. as a consequence of the user waking up) by the auditory stimulation. Furthermore, it provides the user with the option to fall asleep without any auditory stimulation.

The inventors have realized that persons suffering from one or more neurological deficiencies such as mild cognitive impairment, dementia and Alzheimer's disease typically will have difficulties both remembering and carrying out a regularly find it difficult to remember and being able to carry out a repetitive auditory treatment. Therefor it will be a huge advantage of the auditory stimulations can be carried out during sleep.

In fact, according to another embodiment the sound generator 101 is configured to induce neural oscillations that are synchronized, in both frequency and phase with slow wave sleep cycles determined using the EEG monitor 204 and the sleep monitor 205. According to a more specific embodiment this may be achieved using at least one cochlear dispersion compensating chirp adapted to be in phase with a slow wave of slow wave sleep. According to an even more specific embodiment said synchronization (i.e. phase alignment) is made to the upstates of the slow oscillations of slow wave sleep.

There are indications that improvement of slow wave sleep may also alleviate neurological deficiencies such as dementia, Alzheimer's disease and other forms of mild cognitive impairment.

More specifically the scientific paper "Auditory closed-loop stimulation of the sleep slow oscillation enhances memory", by Ngo et. al, in Neuron 78, pages 545 - 553, May 8, 2013 discloses that the auditory stimulation needs to be synchronized with the slow wave sleep, both in phase and frequency, in order to achieve the beneficial effects generally associated with improved slow wave sleep. It is therefore a specific advantage of the present invention that the chirps adapted to compensate the cochlear dispersion can provide a very precise phase synchronization. According to various embodiments the phase synchronization may comprise the use of phase locking or the use of a predetermined time-delay in order to determine when to provide the chirps in response.

It is noted that the different phase synchronization techniques may also be applied to improve the efficiency of inducing other neural oscillations such as the 40 Hz neural oscillations.

Thus, according to an embodiment and referring to Fig. 2 a slow wave phase detector may be incorporated in the audio system 200, being based on input from the EEG monitor 205 and providing as output phase information to the sound generator controller 102 whereby a phase synchronized auditory stimulation signal can be provided.

In an embodiment, an audio system according to the present invention is configured such that two different types of auditory stimulation, both directed at alleviating a neurological deficiency, are provided during sleep. According to a more specific embodiment the first type of auditory stimulation is adapted to induce neural oscillations at a frequency of 40 Hz, while the second type of auditory stimulation is adapted to improve slow wave sleep by providing cochlear dispersion compensated chirps that are repeated with a phase and frequency that is synchronized with the slow wave sleep. Furthermore, the audio system is configured to, based on a determination (which in the following may also be denoted sleep monitoring) of sleep stages, only provide the second type of auditory stimulation when the user is in slow wave sleep, and refrain from providing the first type of auditory stimulation when the user is in slow wave sleep.

Thus, according to more general embodiments of the invention a first type of auditory stimulation is not provided, and a second type of auditory stimulation is only provided when a sleep stage comprising slow waves has been determined.

According to one embodiment the sleep monitoring is carried out using the integrated EEG monitor (204) and a corresponding sleep monitor (205) as already described with reference to Fig. 2.

According to one embodiment the sleep monitoring may be carried out based on an analysis of audio recordings of a user's breathing during sleep, e.g. as described in the paper: "Fine-grained sleep monitoring: Hearing your breathing with smartphones", by Yanzhi Ren et al., presented at the 2015 IEEE Conference on Computer Communications (INFOCOM). According to a preferred embodiment at least one microphone is implemented in the audio system in order to record the user's breathing during sleep. This is advantageous because it enables close proximity audio recording of the user's breathing compared to a less preferred embodiment where the audio system through a wireless link is operationally connected to an external device such as a smart phone in order to receive sleep monitoring results carried out using the smart phone microphone. Furthermore, if two or more microphones are integrated in the audio system various directional audio recording techniques (which may also be denoted beamforming) can be applied to reduce undesired noise (i.e. from a sleeping partner). According to an even more advantageous embodiment the audio system comprises both a first audio device and a second audio device, wherein both devices comprises at least one microphone and a operationally connected using a wireless link such that various binaural directional audio recording techniques can be applied to further improved the quality (i.e. the signal to noise ratio) of the audio recording of the user's breathing during sleep. Finally sleep monitoring based on microphones integrated in an audio system is advantageous because of its simplicity and limited cost.

According to another embodiment the sleep monitoring may be carried out based on analysis of heart rate data as described in the article "Heart rate spectrum analysis for sleep quality detection", by Scherz et al., in Journal of embedded systems 2017, 26 (2017). More specifically the audio system may be operationally connected using a wireless link to an external device (such as a smart phone) capable of providing either the heart rate data or the derived desired sleep data. In a further embodiment a smart phone is configured to receive the heart rate or the derived sleep data from yet another external device such as e.g. a smart watch. However, alternatively a heart rate monitor may be directly integrated in the audio system e.g. in the form of a photoplethysmogram sensor such as a pulse oximeter. This is especially suitable for audio systems adapted to be positioned at least partly in the ear canal of the user.

According to an embodiment the audio system includes an arousal detector based on at least one measured bio-electrical signal such as EEG and Electromyography (EMG). One algorithm for arousal detection based on such signals is presented in the paper: "A computerized algorithm for arousal detection in healthy adults and patients with Parkinson disease", by Soerensen, G.L. et al., in Journal of clinical neurophysiology, 2012 Feb, 29(1) pages: 58-64. Based on such an arousal detector the sound generator controller can be configured to adapt the auditory stimulation level in response to a detected arousal level, whereby, as one example, arousals due to an auditory stimulation can be avoided. However, general monitoring of arousal level during various sleep phases may also be used as a health estimator whereby auditory stimulation can be optimized based on the health estimates provided by the health estimator.

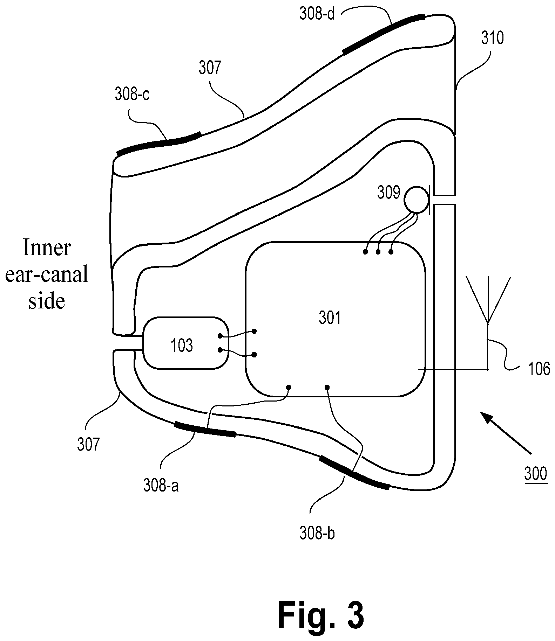

Reference is now made to Fig. 3, which illustrates highly schematically an audio system according to a third embodiment of the invention. The audio system 300 according to the third embodiment primarily distinguishes the audio system 200 in that EEG electrodes 308-a, 308-b, 308-c and 308-d are integrated in the outer walls 307 of the audio system 300. In obvious embodiments it may be selected to include more or fewer EEG electrodes and according to another embodiment no EEG electrodes are included, which may be advantageous with respect to cost.

According to an embodiment the audio system comprises an EEG monitor based on a driven right leg circuit whereby common-mode interference can be reduced.

The audio system 300 is adapted to be positioned in the ear canal of a user. According to an embodiment the shape of the audio system housing is personalized to the ear canal of the individual user based e.g. on rapid prototyping techniques. However, in other embodiments the audio system housing is of the instant-fit type that is designed to be positioned in a great variety of individual ear canals typically by being manufactured in a flexible material. In yet other embodiments the audio system may be adapted to be positioned in the ear, but at least partly outside the ear canal, such as at least partly in the concha or behind the ear or a combination thereof.

Additionally, the audio system 300 comprises a vent 310, which is generally considered advantageous with respect to wearing comfort for the user. According to an embodiment the vent is configured with a variable diameter that is controlled based on input from the sound generator controller, such that the size of the vent diameter is decreased in response to an initiation of the auditory stimulation and to return to normal size in response to a termination of the auditory stimulation. This is advantageous since a decreased vent diameter generally improves the capability of the audio system to provide sound at low frequencies, which may be advantageous for some of the auditory stimulation types considered in the present context. However, a decreased vent diameter is likewise advantageous because it suppresses noise or other disturbing sounds from the sound environment, which may be beneficial with respect to the quality of the provided auditory stimulation. On the other hand it is, as already stated above, generally considered to improve wearing comfort when having a vent that is as open as possible and therefore it is advantageous to increase the vent diameter again when the auditory stimulation is terminated and/or when the user is in a sleep stage that is sufficiently insensitive to possible disturbances and/or when a microphone detects that the background noise from the environment is sufficiently low.

Additionally, the audio system 300 comprises a microphone 309, which, as already discussed above, may be used to record the users breathing sound and hereby derive information about the present sleep stage.

According to an embodiment the audio system 300 that is adapted to be positioned in the ear canal or the user may be configured to include the functionality of a hearing aid, if at least one microphone is likewise included, which is advantageous anyway as discussed above.

Furthermore, the sound generator 101, sound generator controller 102, EEG monitor 204, sleep monitor 205 and a part of the wireless link 205 are integrated in the digital signal processor 301, which is an obvious solution for the skilled person. Finally, it is noted that the components that also are present in some of the previous embodiments have retained their numbering.

According to yet another embodiment, the audio system comprises a first audio device and a second audio device, wherein both devices comprises at least a sound generator, a sound generator controller and a transceiver adapted to operatively connect the two audio devices and wherein said sound generator controller is adapted to synchronize in time the auditory stimulations provided by the two audio devices whereby a diotic presentation of the auditory stimulation is provided.

The inventors have discovered that stronger auditory evoked responses results when binaurally provided auditory stimulations are synchronized in time.

According to an embodiment an audio system comprising both a first and a second audio device, each comprising EEG electrodes, is operationally connected with a galvanic connection between the two audio devices, which is especially advantageous with respect to e.g. sleep monitoring based on EEG signals from both audio devices.

According to another embodiment an audio system comprising both a first and a second audio device, each comprising EEG electrodes, is wirelessly connected. With respect to sleep monitoring based on the EEG signals this audio system is less advantageous than the galvanically connected system, but more advantageous than an audio system consisting of only one audio device.

According to still another embodiment, the audio system comprises an internet link configured to provide an operational connection to at least one remote server. According to a more specific embodiment, the audio system is adapted to: provide measured EEG data, or information derived from the measured EEG data, and corresponding characteristics of at least one provided type of auditory stimulation to said at least one remote server and also adapted to receive at least one optimized characteristic for at least one of said at least one provided type of auditory stimulation from said remote server in response to a request from said audio system or automatically from said remote server based on an analysis of the provided measured EEG data or information derived from the measured EEG data.

Hereby the individual user of an audio system according to the present invention may benefit from the EEG data measured and provided to said remote server from other audio system users. More specifically the remote server may suggest at least one optimized auditory stimulation characteristic based on e.g. machine learning analysis carried out by said remote server. Examples of such an auditory stimulation characteristic includes the specific repetition frequency, the sound level of the auditory stimulation and the parameters defining the chirps adapted to compensate the cochlear dispersion.

According to one specific embodiment the measured EEG data, can be used to evaluate the level (e.g. the energy or Signal-to-Noise-Ratio (SNR)) of induced neural oscillations in the 40 Hz range, which again can be used as an indicator for the progression of cognitive diseases such as Alzheimer's.

According to another specific embodiment the measured EEG data, can be used to estimate the slow wave sleep quality based e.g. on the length or amplitude of the slow wave cycles. Generally, an increased amount of slow wave sleep is considered to reflect an improved health condition.

According to yet another specific embodiment the measured EEG data can be used to estimate the number of arousals and awakenings during sleep, since these incidents are generally considered to indicate a less healthy condition.

However, it is noted that other measured data suitable for evaluating the efficiency of the provided treatment may be used in addition to or instead of EEG data. Examples of such other data includes audio recordings of a user's breathing and heart rate data, both of which can be used to evaluate the quality of sleep.

Thus this type of measured data suitable for evaluating the efficiency of the provided auditory treatment may be denoted health estimates and the means for providing them may be denoted health estimators and the health estimates may be used as feedback in order to optimize the characteristics of the provided auditory stimulation. Thus, according to an embodiment at least one user health estimate provided by at least one user health estimator is used to adapt at least one characteristic of at least one type of auditory stimulation.

It is noted that it is a specific advantage of the present invention that health estimates obtained during sleep are generally considered to of superior quality because a specific user's condition is more stable and less impacted from the many and unpredictable disturbances and specific environmental conditions that typically occur while the user is awake.

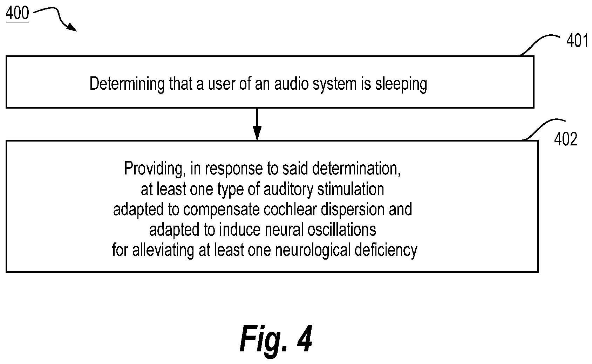

Reference is finally made to Fig. 4, which illustrates highly schematically a method 400 of operating an audio system according to an embodiment of the invention.

According to a first step 401 it is determined that a user of an audio system is sleeping. This may be carried out in a variety of ways as described more detailed above in connection with the audio system embodiments.

According to a second and final step 402 at least one type of auditory stimulation adapted to induce neural oscillations is provided in response to said determination wherein said at least one type of auditory stimulation is adapted to compensate cochlear dispersion, and is adapted to alleviate at least one neurological deficiency.

Hereby is provided an advantageous method of operating an audio system whereby auditory stimulation can be provided without requiring any interaction from the audio system user and additionally providing the auditory stimulation while the audio system user is in a highly receptive state.

Thus an itemized embodiment of the present invention can therefore be expressed as: A method of alleviating a neurological pathology comprising the steps of:

- determining that a user of an audio system is sleeping,
- providing, in response to said determination, at least one type of auditory stimulation adapted to compensate cochlear dispersion and adapted to induce neural oscillations for alleviating at least one neurological deficiency.

In addition to the embodiments discussed with reference to Figs. 1 - 3 the present invention may also be realized as a software application that can be downloaded to an external device such as a smart phone or tablet or similar device whereby both a loudspeaker, a microphone, internet access and abundant processing resources become readily available. According to one specifically advantageous embodiment a separate loudspeaker, such as a headphone set, or earbuds is used to provide the auditory stimulation instead of the built in loudspeaker of the smart phone or tablet. Most advantageously earbuds with a mechanical design directed at optimizing the user comfort when worn during sleep are preferred. Such earbuds may be denoted sleepbuds. It is noted that a headphone set or traditional earbuds may also include EEG electrodes in a manner similar to what has already been discussed e.g. with reference to Fig. 3 and that the measured EEG signals, or information derived therefrom, may be transmitted to the external device for further processing by e.g. a sleep monitor or other type of health estimator.

However, according to an embodiment the audio system does not include a sleep monitor and a first itemized embodiment can therefore be expressed as: An audio system comprising:

- a sound generator configured to provide an auditory stimulation adapted to induce neural oscillations wherein said auditory stimulation is further adapted to compensate cochlear dispersion and to alleviate a neurological pathology; and
- a sound generator controller adapted to at least one of initiate and terminate an auditory stimulation

    wherein the initiation of the auditory stimulation is controlled by a trigger event selected from a group of events comprising: identification of a specific sound environment, a specific time of day, a remotely controlled activation or a user initiated activation and wherein the termination of the auditory stimulation is controlled by a trigger event selected from a group of events comprising: a predetermined time or duration, a detection of speech or own voice or an alarm in the sound environment of the user or a user initiated termination.

More specifically, the user initiated trigger events may be provided by user interaction with the audio system itself or with an associated personal computation device such as a smart phone. It can therefore be seen that the audio system according to the present embodiment is especially advantageous for use while a user is awake.

It is noted that an audio system without a sleep monitor can still be adapted to provide input concerning a detected sleep stage to the sound generator controller, such that at least one of initiation and termination of an auditory stimulation is based on a detected sleep stage, because this information may be obtained from an external sleep monitor wired or preferably wirelessly connected with the audio system using appropriate wired or wireless link means in the audio system.

According to another embodiment the audio system does not include a sound generator adapted to provide an auditory stimulation adapted to compensate cochlear dispersion and a second itemized embodiment can therefore be expressed as: An audio system comprising:

- a sound generator configured to provide an auditory stimulation adapted to induce neural oscillations wherein said auditory stimulation is further adapted to alleviate a neurological pathology;
- a sleep monitor configured to determine at least one stage of sleep; and

- a sound generator controller adapted to at least one of initiate and terminate an auditory stimulation in response to a determined sleep stage.

According to a specific variation of the first and second itemized embodiments the audio system comprises a first audio device and a second audio device, wherein both devices comprises said sound generator, said sound generator controller and a transceiver adapted to operatively connect the two audio devices, and wherein said sound generator controller is adapted to synchronize in time auditory stimulations provided by the two audio devices whereby a diotic presentation of an auditory stimulation can be provided.

According to another specific variation of the first and second itemized embodiments the auditory stimulation is adapted to induce neural oscillations at a frequency of 40 Hz, and according to yet another variation this is carried out using the above mentioned diotic presentation.

It is generally noted that even though many features of the present invention are disclosed in embodiments comprising other features then this does not imply that these features by necessity need to be combined and generally the features of the various embodiments may be freely combined unless it is specifically stated not to be allowed.

As one example the specific characteristics of the auditory stimulation in order to provide cochlear dispersion compensation may be freely combined with the various embodiments related to the determination of a specific user sleep stage. Additionally, the specific characteristics of the auditory stimulation in order to provide cochlear dispersion compensation may also be freely combined with the various purposes of the auditory stimulation such as e.g. inducing neural oscillations of 40 Hz or in the range between 35 - 45 Hz or even in the range between 32 - 48 Hz.

As other examples the use of diotic presentation of an auditory stimulation may be freely combined with any of the disclosed embodiments. As one specific example a diotic presentation may be provided by streaming sound and corresponding control signals from an external device such as a smart phone or tablet and to a set of audio devices (such as earbuds or sleep buds).

Other modifications and variations of the structures and procedures will be evident to those skilled in the art.

## Claims

1. An audio system (200) comprising:

    - a sound generator (101) configured to provide an auditory stimulation adapted to induce neural oscillations wherein said auditory stimulation is further adapted to compensate cochlear dispersion and to alleviate a neurological pathology by:

- - adding a plurality of pure tones each with a specific relative phase, adapted to compensate the cochlear dispersion, and hereby providing a chirp capable of compensating the cochlear dispersion; wherein
- - said specific relative phase is determined using a delay model preferably given by: $\tau = k \cdot f^d$, wherein
$\tau$ is the delay in seconds, f is the frequency in Hz and the constants k and d have the values k = 0.0920 and d = 0.4356; and wherein
- - said plurality of pure tones are separated by a constant frequency difference that is the same as the frequency of the neural oscillations to be induced;

- a sleep monitor (205) configured to determine at least one stage of sleep; and
- a sound generator controller (102) adapted to at least one of initiate and terminate an auditory stimulation in response to a determined sleep stage.

2. The audio system according to claim 1, wherein the specific relative phases depend on the auditory stimulation level.

3. The audio system according to claim 1, wherein a first type of auditory stimulation is adapted to induce neural oscillations at a frequency of 40 Hz.

4. The audio system according to claim 1, wherein a second type of auditory stimulation is provided by at least one chirp adapted to be in phase with a slow wave of slow wave sleep.

5. The audio system according to claim 1 further comprising an EEG monitor (204) adapted to at least one of: measure a level of induced neural oscillations at 40 Hz, to measure a level of slow wave sleep, to measure EEG data for determining at least one stage of sleep, and to measure EEG data for evaluating the efficiency with respect to alleviating a neurological pathology.

6. The audio system according to claim 1 further comprising:

- an arousal detector based on a measured bioelectrical signal selected from a group of bioelectrical signals comprising EEG and EMG signals; and wherein the sound generator controller (102) is configured to adapt the auditory stimulation level in response to a detected arousal level.

7. The audio system according to claim 1, comprising a user health estimator, wherein the sound generator controller (102) is configured to adapt at least one characteristic of at least one type of auditory stimulation based on at least one user health estimate provided by the user health estimator.

8. The audio system according to claim 1, wherein a first type of auditory stimulation is not provided and a second type of auditory stimulation is only provided when a sleep stage comprising slow waves has been determined.

9. The audio system according to claim 1 comprising a first audio device and a second audio device, wherein both devices comprises said sound generator, said sound generator controller, a transceiver adapted to operatively connect the two audio devices, and wherein said sound generator controller is adapted to synchronize in time the auditory stimulations provided by the two audio devices, whereby a diotic presentation of the auditory stimulation is provided.

**Patentansprüche**

1. Audiosystem (200), umfassend:

- einen Tongenerator (101), der konfiguriert ist, um eine auditive Stimulation bereitzustellen, die angepasst ist, um neuronale Oszillationen zu induzieren, wobei die auditive Stimulation weiter angepasst ist, um Kochlear-Dispersion zu kompensieren und um eine neurologische Pathologie zu lindern durch:

-- Hinzufügen einer Vielzahl von reinen Tönen, jeweils mit einer bestimmten relativen Phase, die angepasst sind, die Kochlear-Dispersion zu kompensieren, und dadurch Bereitstellen eines Piepens, das imstande ist, die Kochlear-Dispersion zu kompensieren; wobei
-- die bestimmte relative Phase unter Verwendung eines Verzögerungsmodells bestimmt wird, das vorzugsweise ist durch: $\tau = k \cdot f^d$, wobei
$\tau$ die Verzögerung in Sekunden ist, f die Frequenz in Hz ist und die Konstanten k und d die Werte k = 0,0920 und d = 0,4356 aufweisen; und wobei

- die Vielzahl der reinen Töne durch einen konstanten Frequenzunterschied getrennt ist, der der gleiche ist, wie die Frequenz der zu induzierenden neuronalen Oszillationen;
- einen Schlafmonitor (205), der konfiguriert ist, um mindestens eine Schlafphase zu bestimmen; und
- eine Tongenerator-Steuereinheit (102), die angepasst ist, um als Reaktion auf eine bestimmte Schlafphase mindestens eines zu tun, von eine auditive Stimulation einleiten und beenden.

2. Audiosystem nach Anspruch 1, wobei die bestimmten relativen Phasen von dem auditiven Stimulationspegel abhängen.

3. Audiosystem nach Anspruch 1, wobei ein erster Typ von auditiver Stimulation angepasst ist, um neuronale Oszillationen mit einer Frequenz von 40 Hz zu induzieren.

4. Audiosystem nach Anspruch 1, wobei ein zweiter Typ von auditiver Stimulation durch mindestens ein Piepen bereitgestellt wird, das angepasst ist, mit einer langsamen Welle von Slow-Wave-Schlaf phasengleich zu sein.

5. Audiosystem nach Anspruch 1, das weiter einen EEG-Monitor (204) umfasst, der angepasst ist, um mindestens eines zu tun von: einen Pegel induzierter neuronaler Oszillationen bei 40 Hz zu messen, einen Slow-Wave-Schlafpegel zu messen, EEG-Daten zum Bestimmen mindestens einer Schlafphase zu messen, und EEG-Daten zum Bewerten der Effizienz in Bezug auf Linderung einer neurologischen Pathologie zu messen.

6. Audiosystem nach Anspruch 1, weiter umfassend:

- einen Erregungsdetektor basierend auf einem gemessenen bioelektrischen Signal, das aus einer Gruppe bioelektrischer Signale ausgewählt ist, die EEG- und EMG-Signale umfasst; und

wobei die Tongenerator-Steuereinheit (102) konfiguriert ist, um den auditiven Stimulationspegel als Reaktion auf einen detektierten Erregungspegel anzupassen.

7. Audiosystem nach Anspruch 1, das einen Benutzergesundheitsschätzer umfasst,
wobei die Tongenerator-Steuereinheit (102) konfiguriert ist, um mindestens eine Eigenschaft mindestens eines Typs von auditiver Stimulation basierend auf mindestens einer vom Benutzergesundheitsschätzer bereitgestellten Benutzergesundheitsschätzung anzupassen.

8. Audiosystem nach Anspruch 1, wobei ein erster Typ von auditiver Stimulation nicht bereitgestellt wird und ein zweiter Typ von auditiver Stimulation nur bereitgestellt wird, wenn eine Schlafphase bestimmt wurde, die langsame Wellen umfasst.

9. Audiosystem nach Anspruch 1, das eine erste Audiovorrichtung und eine zweite Audiovorrichtung umfasst, wobei beide Vorrichtungen den Tongenerator, die Tongenerator-Steuereinheit und einen Sendeempfänger, der angepasst ist, um die beiden Audiovorrichtungen wirkzuverbinden, umfassen, und wobei die Tongenerator-Steuereinheit angepasst ist, um die von den beiden Audiovorrichtungen bereitgestellten auditiven Stimulationen zeitlich zu synchronisieren, wodurch eine diotische Präsentation der auditiven Stimulation bereitgestellt wird.

## Revendications

1. Système audio (200) comprenant :

- un générateur de son (101) configuré pour fournir une stimulation auditive conçue pour induire des oscillations neuronales, dans lequel ladite stimulation auditive est en outre conçue pour compenser la dispersion cochléaire et pour soulager une pathologie neurologique par :

-- ajout d'une pluralité de sons purs, chacun avec une phase relative spécifique, conçus pour compenser la dispersion cochléaire, et fournissant ainsi une fluctuation de longueur d'onde apte à compenser la dispersion cochléaire ; dans lequel
-- ladite phase relative spécifique est déterminée en utilisant un modèle de retard donné de préférence par : $\tau = k \cdot f^d$, dans lequel $\tau$ est le retard en secondes, f est la fréquence en Hz et les constantes k et d présentent les valeurs k = 0,0920 et d = 0,4356 ; et dans lequel
-- ladite pluralité de sons purs sont séparés par une différence de fréquence constante qui est la même que la fréquence des oscillations neuronales à induire ;

- un moniteur de sommeil (205) configuré pour déterminer au moins un stade de sommeil ; et
- un dispositif de commande de générateur de son (102) conçu pour au moins l'un parmi le lancement et l'arrêt d'une stimulation auditive en réponse à un stade de sommeil déterminé.

2. Système audio selon la revendication 1, dans lequel les phases relatives spécifiques dépendent du niveau de stimulation auditive.

3. Système audio selon la revendication 1, dans lequel un premier type de stimulation auditive est conçu pour induire des oscillations neuronales à une fréquence de 40 Hz.

4. Système audio selon la revendication 1, dans lequel un second type de stimulation auditive est fourni par au moins une fluctuation de longueur d'onde conçue pour être en phase avec une onde lente du sommeil lent profond.

5. Système audio selon la revendication 1 comprenant en outre un moniteur EEG (204) conçu pour au moins l'une parmi : la mesure d'un niveau d'oscillations neuronales induites à 40 Hz, la mesure d'un niveau de sommeil lent profond, la mesure de données EEG pour déterminer au moins un stade de sommeil, et la mesure de données EEG pour évaluer l'efficacité par rapport au soulagement d'une pathologie neurologique.

6. Système audio selon la revendication 1, comprenant en outre :

   - un détecteur d'éveil basé sur un signal bioélectrique mesuré sélectionné à partir d'un groupe de signaux bioélectriques comprenant des signaux EEG et EMG ; et

   dans lequel le dispositif de commande de générateur de son (102) est configuré pour adapter le niveau de stimulation auditive en réponse à un niveau d'éveil détecté.

7. Système audio selon la revendication 1, comprenant un estimateur de santé d'utilisateur,
   dans lequel le dispositif de commande de générateur de son (102) est configuré pour adapter au moins une caractéristique d'au moins un type de stimulation auditive sur la base d'au moins une estimation de santé d'utilisateur fournie par l'estimateur de santé d'utilisateur.

8. Système audio selon la revendication 1, dans lequel un premier type de stimulation auditive n'est pas fourni et un second type de stimulation auditive est fourni uniquement lorsqu'un stade de sommeil comprenant des ondes lentes a été déterminé.

9. Système audio selon la revendication 1 comprenant un premier dispositif audio et un second dispositif audio, dans lequel les deux dispositifs comprennent ledit générateur de son, ledit dispositif de commande de générateur de son, un émetteur-récepteur conçu pour connecter fonctionnellement les deux dispositifs audio, et dans lequel ledit dispositif de commande de générateur de son est conçu pour synchroniser dans le temps les stimulations auditives fournies par les deux dispositifs audio, selon lequel une présentation diotique de la stimulation auditive est fournie.

**Fig. 1**

**Fig. 2**

**Fig. 3**

400

401

Determining that a user of an audio system is sleeping

402

Providing, in response to said determination,
at least one type of auditory stimulation
adapted to compensate cochlear dispersion and
adapted to induce neural oscillations
for alleviating at least one neurological deficiency

## Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014162286 A1
- US 2019388020 A1
- US 2013190556 A1
- JP 2011083393 A

### Non-patent literature cited in the description

- **MCDERMOTT et al.** Gamma Band Neural Stimulation in Humans and the Promise of a New Modality to Prevent and Treat Alzheimer's Disease. *Journal of Alzheimer's Disease,* 2018, vol. 65, 363-392
- **ARTIEDA et al.** Potentials evoked by chirp-modulated tones: a new technique to evaluate oscillatory activity in the auditory pathway. *Clinical Neurophysiology,* 2004, vol. 115, 699-709
- **DAU et al.** Auditory brainstem responses with optimized chirp signals. *Journal of Acoustical Society America,* 2000, vol. 107, 1530-1540
- **ELBERLING et al.** Auditory steady-state responses to chirp stimuli based on cochlear traveling. *Journal of Acoustical Society America,* vol. 122, 2772-2785
- **ELBERLING et al.** A direct approach for the design of chirp stimuli used for the recording of auditory brainstem responses. *Journal of Acoustical Society America,* vol. 128, 2955-2964
- **NGO.** Auditory closed-loop stimulation of the sleep slow oscillation enhances memory. *Neuron,* 08 May 2013, vol. 78, 545-553
- **YANZHI REN et al.** Fine-grained sleep monitoring: Hearing your breathing with smartphones. *2015 IEEE Conference on Computer Communications (INFOCOM)*
- **SCHERZ et al.** Heart rate spectrum analysis for sleep quality detection. *Journal of embedded systems,* 2017, vol. 2017, 26
- **SOERENSEN, G.L. et al.** A computerized algorithm for arousal detection in healthy adults and patients with Parkinson disease. *Journal of clinical neurophysiology,* February 2012, vol. 29 (1), 58-64